# EUROPEAN PATENT APPLICATION

(11) **EP 4 455 271 A1**
(43) Date of publication of application: **30.10.2024**
(21) Application number: 23170502.1
(22) Date of filing: 27.04.2023
(51) Int. Cl.: C12N 5/071

(54) **METHOD FOR PRODUCING MILK LIKE PRODUCTS**

(71) Applicant: Société des Produits Nestlé S.A., 1800 Vevey (CH)
(72) Inventor:
(74) Representative: CMS Cameron McKenna Nabarro Olswang LLP

(57) **Abstract**

A method of producing mammalian breast milk exosomes, for example human breast milk exosomes comprising generating lactocytes derived from mammalian mammary epithelial cells, for example human mammary epithelial cells, expressing the mammalian milk like product, for example the human milk like product from lactocytes, and purifying the exosomes from the mammalian milk like product.

## Description

### Field of the invention

The present invention concerns *in vitro* methods for producing isolated mammalian breast milk exosomes, the method comprising generating lactocytes, derived from mammalian mammary epithelial cells, for example human mammary epithelial cells, through culture and differentiation, and/or mammary-like gland organoids comprising such lactocytes, expressing a mammalian milk like product, for example the human milk like product from such lactocytes and/or mammary-like gland organoids, and purifying the exosomes from the mammalian milk like product. The present invention also relates to the isolated mammalian breast milk exosome obtainable from such method.

### Background of the invention

Mammalian and especially human milk is a complex fluid with a multitude of components, each of which may contribute substantially to infant and perhaps maternal health. It is becoming increasingly clear that human breastmilk is the most appropriate source of nutrition at least up to the age of 6 months. Many components of human milk are simply not found or poorly found or less active in cow's milk upon which infant formula manufacture is based. This includes for instance protein lactoferrin, growth factors, long chain polyunsaturated fatty acids or oligosaccharides. Human milk composition has been used as a gold standard to develop current infant formula, despite recent major development in infant formula composition, it is illusory to think that human milk replication or replication of components of said human milk will be achieved with current manufacturing processes.

Today the only source of human milk is human donors (breastfeeding mothers). Donation are reported for non-commercial use (human milk biobank) and commercial use. However, this is limited and has strong regulatory, safety and sometime ethical or religious constraints.

Stem cells were found in mammalian and especially human milk called human breastmilk stem cells (hBSC). hBSCs were shown to be highly plastic and to differentiate in culture into multiple cell types and more importantly into the three lineages required to shape the lobulo-alveolar structure of the human mammary gland (Hassiotou F. et al. Stem Cells. 2012). However, the use of hBSC to produce human breast milk is neither practical nor sustainable, as it requires human donors.

A technology based on a cell line with stem cell functionality - called induced pluripotent stem cells (iPSC) is known. A reliable two step protocol to generate human mammary like organoids from human iPSC (hiPSC) was developed (Ying Qu et al, Stem Cell Report vol 8, 205-215, February 14th 2017).

Accordingly, it is an object of the present invention to provide alternative and improved methods for producing mammalian milk and key components of mammalian milk such as exosomes using cultured cells, without the use of early stage undifferentiated stem cells. It is also an object of the present invention to prepare customized mammalian milk like product, for example human milk like product, in cultured cells which could be adapted to specific needs of the recipient and/or to produce human milk bioactives such as exosomes to complement existing cow-based solutions for infant nutrition, again without the use of stem cells.

### Summary of the invention

The present invention solves the above-mentioned technical problem.

Provided herein is an *in vitro* method for producing mammalian breast milk exosomes, the method comprising:
A) Culturing mammary epithelial cells in a culture medium to generate lactocyte mammary-like gland organoids; and
B) Secreting a mammalian milk like product from said lactocytes, and
C) Purifying the exosomes from the mammalian milk like product to remove impurities, optionally by chromatography or filtration or ultracentrifugation, in order to isolate the exosomes.

Also provided herein is a mammary breast milk exosome product which is obtainable according to the method as described anywhere herein, optionally a human breast milk exosome.

Finally, provided herein is a method of producing a mammalian milk fortifier, comprising conducting the method as described anywhere herein.

### Detailed description of the invention

### Definitions

Within the context of the present invention, the term "in vitro" means performed or taking place in a test tube, culture dish, bioreactor or elsewhere outside a living organism.

Within the context of the present invention, the term "mammalian" identify an animal belonging to the mammalian species, for example human, cow, monkey, camel, sheep, goat etc.

Within the context of the present invention, the terms "lactocytes" or "mammary-like cells" identify secretory epithelial cells expressing CK18 cell marker and derived from mammalian mammary epithelial cells and in particular from human mammary epithelial cells. Human mammary epithelial cells as used herein are commercially available and may be selected from any suitable cell line. A suitable human mammary epithelial cell line in the context of the current invention is e.g., a non-tumorigenic cell line such as MCF-10, or may be a tumorigenic cell line such as MCF-7.

Within the context of the present invention the terms "mammary gland like organoids" or "mammary like organoids" mean a miniaturized and simplified version of a mammary gland which develops in two or three dimensions (2D/3D) and which comprises lactocytes as above defined.

Within the context of the present invention, the term "human milk like product" is a cell cultured milk product. It is an edible product which is expressed by the lactocytes and/or mammary gland like organoids generated according to the process of the present invention.

The "human milk like product" according to the present invention can have the same components as human breast milk of a well-nourished mother (for example in terms of bioactives, macro and micronutrients and levels thereof). This is referred to herein as a "standard human milk product". Alternatively, "human milk like product" according to the present invention can have altered ratios and concentrations of components found naturally in human breast milk of a well-nourished mother. This is referred to herein as a "non-standard milk like product". A "human milk like product" according to the present invention can be modified such that it includes components that are not found naturally in human breast milk of a well-nourished mother (a "modified milk like product"). Non-limiting examples of human milk like products are selected from the group consisting of: supplement, fortifier, human breast milk substitute (or replacer) and ingredient enriched in only one and/or a portion of bioactives, macro- and micronutrients which can be typically found in human breast milk of a well-nourished mother.

The "human milk like product" can be used to replace consumption of naturally lactated milk (a "human milk substitute"). The milk substitute product can be used as a supplement (a "human milk supplement") or as a fortifier (a "human milk fortifier") to be consumed in combination with naturally lactated milk.

In an embodiment, the standard human milk like product according to the present invention comprises at least macro- and micronutrients which can be typically found in human breast milk of a well-nourished mother. In one embodiment, the human milk like product according to the present invention comprises: proteins, peptides, lipids (including linoleic acid and alpha-linolenic acid), carbohydrates, Vitamins (including Vitamin A, Vitamin D3, Vitamin E, Vitamin K, Thiamin, Riboflavin, Niacin, Vitamin B6, Vitamin B12, Pantothenic acid, folic acid, Vitamin C and Biotin), minerals (including iron, calcium, phosphorus, magnesium, sodium, chloride, potassium, manganese, iodine, selenium, copper and zinc), choline, myoinositol and L-carnitine. In one embodiment, the human milk like product according to the present invention also comprises at least one bioactive selected from the group consisting of: growth factors, cytokines, probiotics, extracellular vesicles (e.g. milk fat globules and or exosomes), bioactives from exosome (for example miRNA) and secretory IgA.The standard human milk like product according to the present invention is not the product of human breast lactation as occurring in nature.

In another embodiment, the human milk like product according to the present invention can be adapted to specific needs of the infant who will receive it. It may comprise only one and/or a portion of bioactives, macro and micro nutrients which can be typically found in human breast milk of a well-nourished mother. In such embodiment, the human breast milk like product may also be referred to with the term "non-standard human milk like product". In one embodiment, the non-standard human milk like product according to the present invention comprises one or more of the nutrients or bioactives selected from the group consisting of proteins, peptides, lipids (including linoleic acid and alpha-linolenic acid), carbohydrates (including human milk oligosaccharides), Vitamins (including Vitamin A, Vitamin D3, Vitamin E, Vitamin K, Thiamin, Riboflavin, Niacin, Vitamin B6, Vitamin B12, Pantothenic acid, folic acid, Vitamin C and Biotin), minerals (including iron, calcium, phosphorus, magnesium, sodium, chloride, potassium, manganese, iodine, selenium, copper and zinc), choline, myoinositol, L-carnitine, growth factors, cytokines, probiotics, extracellular vesicles (e.g. milk fat globules and or exosomes), bioactives from exosome (for example miRNA) and secretory IgA.

Within the context of the present invention, the term "non-modified human milk like product" indicates a human milk like product which is expressed by lactocytes and/or by the mammary gland like organoids generated according to steps A) and B) of the process of the present invention and which is not subject to the further treatment according to optional step C) of the process of present invention. Non-modified human milk like product may comprise both standard and non-standard human milk like products. Non limiting examples of non-standard human milk like products are selected from the group consisting of: supplement, fortifier, and ingredient enriched in only one and/or a portion of bioactives, macro and micro nutrients which can be typically found in human breast milk of a well-nourished mother.

Within the context of the present invention, the term "modified human milk like product" indicates a human milk like product which is expressed by lactocytes and/or by the mammary gland like organoids generated according to steps A) and B) of the process of the present invention and which is subject to the further treatment according to optional step C) of the process of present invention.

Modified human milk like products may comprise both standard and non-standard human milk like products.

Within the context of the present invention the term "EBs" means "embryoid bodies".

Within the context of the present invention the term "mEBs" means "MammoCult medium-cultured embryoid bodies".

MammoCult medium refers to a serum-free culture medium comprising basal medium, at least one proliferation supplement, heparin and hydrocortisone.

Within the context of the present invention the terms "embryoid bodies (EBs)", "MammoCult medium-cultured embryoid bodies (mEBs)", "mammospheres" and/or "spheroids" refer to three-dimensional aggregates formed in suspension under step A) of the process of the present invention.

The term "infant" in the context of the present invention identifies a child under the age of 12 months, such as under the age of 9 months, particularly under the age of 6 months.

In the context of the present invention the infant may be any term infant or preterm infant. In an embodiment of the invention, the infant is selected from the group of preterm infants and term infants.

The term "term infant" refers to infants born at term or at a gestational age of 37 weeks or more.

The term "preterm infant" refers to infants who are born at a gestational age of less than 37 weeks.

In the context of the present invention, the term "birth weight" means the first weight of the fetus or newborn obtained after birth.

Within the context of the present invention, the term "low birth weight" means a birth weight of less than 2500 g (up to and including 2499 g).

Within the context of the present invention, the term "very low birth weight" means a birth weight of less than 1500 g (up to and including 1499 g).

Within the context of the present invention, the term "extremely low birth weight" means a birth weight of less than 1000 g (up to and including 999 g).

The term "small for gestational age infant" refers to infants having a birth weight that is more than 2 standard deviations below the mean reference to a birth weight for gestational growth chart or having a birth weight that is less than the 10^{th} percentile of population-based weight data obtained from infants at the same gestational age. The term "small for gestational age infants" includes infants who are small at birth either from a constitutive or genetic origin or, as a consequence of intrauterine growth restriction.

Within the context of the present invention, the term "young children" or "toddler" indicates a child between the age of 1 and 3 years.

The term "infant formula" as used herein refers to a nutritional composition intended for infants and as defined in *Codex Alimentarius,* (Codex STAN 72-1981) and Infant Specialities (incl. Food for Special Medical Purpose) as defined in *Codex Alimentarius,* (Codex STAN 72-1981). It also refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). The infant formulas encompass the starter infant formulas and the follow-up or follow-on formulas. Generally, a starter formula is for infants from birth as breast-milk substitute, and a follow-up or follow-on formula from the 6th month onwards.

The "growing-up milks" (or GUMs) are given from one year onwards. It is generally a milk-based beverage adapted for the specific nutritional needs of young children. They are nutritional compositions used for feeding children from 12 months to 2-3 years old in combination with other foods.

Within the context of the present invention, the term "fortifier"" refers to a composition which comprises one or more nutrients having a nutritional benefit for infants or young children.

By the term "milk fortifier", it is meant any composition used to fortify or supplement either human breast milk, infant formula, growing-up milk or human breast milk fortified with other nutrients. Accordingly, the human milk fortifier of the present invention can be administered after dissolution in human breast milk, infant formula, growing-up milk or human breast milk fortified with other nutrients or otherwise it can be administered as a standalone composition.

When administered as a stand-alone composition, the human milk fortifier of the present invention can be also identified as being a "supplement". In one embodiment, the milk fortifier of the present invention is a supplement.

By the term "human milk fortifier", it is meant any composition used to fortify or supplement human breast milk, or human breast milk fortified with other nutrients. The "human milk fortifier" according to the present invention may be intended to be administered to infants who were born preterm, with very low birth weight (VLBW) or with extremely low birth weight (ELBW).

The milk fortifier according to the present invention may be in powder or liquid form.

Milk fortifier compositions having a liquid form presents some particular benefits. For example, liquid formulations might be more convenient if coupled with a packaging that delivers calibrated drops of a certain weight or volume.

In addition, liquid formulations are easier to mix with the compositions to be fortified, whereas the powder ones can, in some cases, form lumps.

Within the context of the present invention, the term "nutritional composition" means a composition which nourishes a subject. This nutritional composition is usually to be taken orally or intravenously. It may include a lipid or fat source, a carbohydrate source and/or a protein source. In a particular embodiment the nutritional composition is a ready-to-drink composition such as a ready-to-drink formula.

Reference herein to EpiCult or EpiCultB medium refers to a serum free culture medium comprising hydrocortisone, insulin, FGF10 and HGF.

A culture medium as disclosed anywhere herein refers to a solid, semi-solid or liquid comprising essential nutrients, designed to support the growth and differentiation of microorganisms. MammoCult medium is one example of a culture medium that may be used in the present invention.

### Methods and Uses

The present invention relates to methods of producing mammary gland cells using mammalian epithelial cells that are cultured in specific conditions described below and using said mammary gland cells for producing a mammalian milk like product in vitro, wherein exosomes are purified from the mammalian milk like product in order to isolate the exosomes.

It has been surprisingly demonstrated in the present invention that mammary epithelial cells can be used as the starting material in a protocol for producing a mammalian milk like product. Using mammary epithelial cells as a starting material means that there is no need for a complicated differentiation protocol to first produce the mammary epithelial cells from e.g., early stage undifferentiated stem cells. This reduces the overall time of the method for producing a mammalian milk like product compared to stem cell protocols, and has cost saving benefits.

Thus, the present invention relates to a method for producing a mammalian milk like product, comprising:
A) Culturing mammary epithelial cells in a culture medium to generate lactocyte mammary-like gland organoids; and
B) Secreting the mammalian milk like product from said lactocytes.

### Mammalian milk like product production

The present invention relates to methods for producing a mammalian milk like product as defined herein, including any of steps A) and B) as defined herein and optional step C) as defined herein.

### Step A - Generating lactocytes and/or mammary like organoids

According to the method of the present invention, mammary like cells and/or organoid structures are generated under step A).

This includes the proliferation and maturation the mammary epithelial cells to produce mammary like organoids, such as lactocytes.

In the methods disclosed herein, mammary epithelial cells are used as the starting material, i.e., culturing of the epithelial cells is the first step of the method.

The proliferation and maturation of mammary epithelial cells occurs by culturing the mammary epithelial cells in specific culture medium, for example complete MammoCult medium (StemCell Technologies). Complete MammoCult medium is preferably composed of the basal medium, proliferation supplements, heparin (typically 4µg/mL), and hydrocortisone (typically 0.48µg/mL). Medium is usually changed every three days. mEBs (mammospheres) obtained in said step are then enriched for non-neural ectoderm cells.

In some embodiments, the proliferation and maturation stage is between day 0 and day 7, where day 0 is the time point where the mammary epithelial cells are first added to the culture medium. In some embodiments, the proliferation and maturation stage is for 7 days. In some embodiments, the proliferation and maturation stage is for no longer than 7 days.

After the proliferation and maturation stage, the cells are induced to express milk proteins. In some embodiments, said induction period is between day 7 and day 14. In some embodiments, the induction period is for 7 days. In some embodiments, the induction period is for no longer than 7 days.

In one embodiment of the present invention, a method for producing a human milk like product is provided comprising generating lactocytes under step A) from human mammary epithelial cells, where such step A) comprises:
i) culturing human mammary epithelial cells in an appropriate culture medium (for example MammoCult medium), and after 7 days generating lactocytes.

In another embodiment, a method for producing a human milk like product is provided comprising generating lactocytes under step A) from human mammary epithelial cells, where such step A) comprises:
i) culturing human mammary epithelial cells in an appropriate culture medium (for example MammoCult medium), in non-adherent conditions for at least 7 days to generate lactocytes.

In one embodiment, the method according to the present invention provides for culture conditions according to step A), which are adapted to generate lactocytes derived from human mammary epithelial cell capable to secrete a human milk like product.

In a preferred embodiment, a method for producing a human milk substitute product is provided comprising generating lactocytes under step A) from human mammary epithelial cells, where such step A) comprises proliferating and maturing mammary epithelial cells to differentiate towards mammary gland cells (for example lactocytes) in an appropriate 3D culture system as described anywhere herein (for example 3D-suspension condition). In some embodiments, for at least 7 days. In some embodiments, for 7 days or less.

In another preferred embodiment, a method for producing a human milk like product is provided comprising generating lactocytes under step A) from human mammary epithelial cells, where such step A) comprises:
i) culturing mammary epithelial cells in an appropriate culture medium (for example MammoCult medium), in an appropriate 3D culture system (for example 3D-suspension condition) for at least 7 days (day 0 to day 7), , to generate lactocytes.

In a particularly preferred embodiment of the present invention, a method of producing a human milk like product is provided comprising generating lactocytes under step A) from human mammary epithelial cells, wherein:
i) culturing mammary epithelial cells in complete MammoCult medium (StemCell Technologies) comprising the basal medium, proliferation supplement and supplemented with heparin (typically 4µg/mL), hydrocortisone (typically 0.48µg/mL), for 7 days (day 0-day 7), and
ii) induction of milk protein expression by incubating the cells in EpiCultB medium supplemented with EpiCult proliferation supplement, hydrocortisone, insulin, FBS, prolactin, progesterone and β-estradiol for 7 days (day 7-day 14).

Step ii) preferably leads to differentiation into milk protein expressing cells, particularly lactocytes, and/or mammary like gland organoids.

In a further particularly preferred embodiment of the present invention, a method of producing a human milk like product is provided comprising generating lactocytes under step A) from human mammary epithelial cells, wherein:
i) culturing mammary epithelial cells in MammoCultB medium supplemented with MammoCult proliferation supplement, hydrocortisone, heparin as described anywhere herein, for 7 days (day 0-day 7), and
ii) induction of milk protein expression by incubating the cells in EpiCultB medium supplemented with EpiCult proliferation supplement, hydrocortisone, insulin, FBS, prolactin, progesterone and β-estradiol for 7 days (day 7 to day 14).

Step ii) preferably leads to differentiation into milk protein expressing cells, particularly lactocytes, and/or mammary like gland organoids.

In one embodiment, steps ii) as defined above for the particularly preferred embodiments, preferably lead to formation of/differentiation into at least breast cells, luminal cells, and basal cells. In this context, breast cells preferably express one or more, preferably all of markers selected from the group consisting of: β-Casein, milk protein, and hormone receptors. Moreover, luminal cells preferably express one or more, preferably all markers selected from the group consisting of: EpCAM, MUC1, CD49F, GATA3, CK8, and CK18. Moreover, basal cells preferably express one or more markers selected from the group consisting of: CK14, α-smooth muscle actin and P63.

In one further embodiment, after step ii) as defined above for the particularly preferred embodiments, mammary like gland organoids may be obtained, that express one or more markers selected from the group consisting of: β-Casein, milk protein, and hormone receptors, luminal cells that express one or more markers selected from the group consisting of: EpCAM, MUC1, CD49F, GATA3, CK8, CK18, and basal cells that express one or more markers selected from the group consisting of: CK14, α-smoothmuscle actin and P63.

In one embodiment of the invention, the methods above described are provided for producing a human milk like product.

In one embodiment (of step A), delivery of nutrients and biomimetic stimuli is controlled to influence cell growth, differentiation and tissue formation. In one embodiment (of step A), such control is performed in a bioreactor.

In some embodiments, the mammary like gland organoids or lactocytes derived from Step A have high expression of mammary gland specific markers. In some embodiments, the mammary like gland organoids or lactocytes derived from Step A express Keratin 18 (KRT-18). In some embodiments, the mammary like gland organoids or lactocytes derived from Step A express estrogen receptor (ER). In some embodiments, mammary like gland organoids or lactocytes derived from Step A express more than 59% of KRT-18 and ER, as estrogen-receptor-positive luminal mammary gland population.

In some embodiments, the mammary like gland organoids or lactocytes derived from Step A have high expression of key milk bioactives. In some embodiments, the mammary like gland organoids or lactocytes derived from Step A have increased expression of key milk bioactives. In some embodiments, the mammary like gland organoids or lactocytes derived from Step A have increased expression of key milk bioactives post-induction (for example at day 14) compared to pre-induction (for example at day 7). In some embodiments, the mammary like gland organoids or lactocytes derived from Step A have increased mRNA expression of lactoferrin (LTF) post-induction compared to pre-induction. In some embodiments, the mammary like gland organoids or lactocytes derived from Step A have increased mRNA expression of milk fat globule-EGF factor 8 (MFGE8) post-induction compared to pre-induction.

It will be understood that any method or method step disclosed herein may be conducted in 3D suspension culture rather than using a membrane matrix as a support. Thus, in some embodiments, during all the differentiation procedure, cells are maintained in suspension culture.

### Step B - Expressing a human breast milk like product

In one embodiment of the present invention, the methods comprise expressing the human milk like product from mammary like organoids derived from human mammary epithelial cells, preferably prepared according to step A). Expressing human milk like products preferably occurs upon induction of expression of the human milk like product from such lactocytes and/or mammary-like gland organoids.

In one embodiment, lactating lactocytes are induced by applying a specific medium (for example EpiCultB) supplemented with lactogenic factors (for example prolactin, hydrocortisone, and insulin).

Particularly, the human milk like product obtained from mammary like organoids derived from human mammary epithelial cells, preferably prepared according to step A), contains bioactives of human milk, selected from the group comprising or consisting of proteins, lipids or oligosaccharides, preferably human milk oligosaccharides, etc.. Inventors particularly managed to identify with the particularly preferred protocol according to steps A i) to iv) as carried out above inter alia oligosaccharides (including lactose and some HMOs), lipids (including 4 fatty acids), proteins (7 detected including caseins), and miRNA (75 detected, including 11 typically detected in HBM).

In one embodiment, the human milk like product obtained from mammary like organoids derived from human mammary epithelial cells, preferably prepared according to step A), contains bioactives of human milk, selected from the group comprising or consisting of: oligosaccharides, lipids, proteins, exosomes and miRNA. In one embodiment, the human milk like product obtained from mammary like organoids derived from human induced pluripotent stem cells (hiPSCs), preferably prepared according to step A), contains exosomes.

In another embodiment, human milk like product obtained from mammary like organoids derived from human mammary epithelial cells, preferably prepared according to step A), contains bioactives of human milk, selected from the group comprising or consisting of: lactose, 6'SL, C-4:0 fatty acid, C-8:0 fatty acid, C-10:0 fatty acid, C-14:0 fatty acid, C-15:0 fatty acid, C-16:0 fatty acid, C-16:1n7 fatty acid, C-17:0 fatty acid, C-18:0 fatty acid, C-18:1 n9 fatty acid, C-18:1 fatty acid, C-18:2 n6 fatty acid, C-20:0 fatty acid, C-20:1 n9 fatty acid, C-18:3 n3 fatty acid, C-22:0 fatty acid, lactoferrin, albumin, prolactin, Alpha S1-casein, Hemoglobin subunit beta, Hemoglobin subunit alpha, α-lactalbumin, Alpha-2-macroglobulin, β-casein, bile salt-activated lipase, κ-casein, lactadherin, CD14, fatty acid synthase, IgA, plgR, Serum albumin, Xanthine dehydrogenase, exosomes, miR-21-5p, miR-181a-5p, miR-30d-5p, miR-30b-5p, miR-22-3p, miR-146b-3p, miR-30c-5p, miR-30a-5p, miR-30e-5p and miR-148b-3p.

In one embodiment of the present invention, the human milk like product obtained from mammary like organoids derived human mammary epithelial cells is a standard human milk product. In another embodiment of the present invention, the human milk like product obtained from mammary like organoids derived from human mammary epithelial cells, is a non-standard human milk product.

### Step C - Further treatments to produce modified human breast milk like product

In one optional embodiment of the present invention, the herein-described methods comprise an additional step C) which is performed on the human milk like product obtainable from step B) and which comprises performing an additional treatment on such product to provide a modified human milk like product.

In one particular embodiment, the additional treatment step C) performed on the inventive human breast milk like product may be selected from the group consisting of: a purification step, an isolation process, an extraction process, a fractionation step, an enrichment process, an enzymatic treatment, the addition of further components (for example which can't be expressed by the human mammary gland organoid (such as for example Immunoglobulins, probiotic and/or minerals) or combinations thereof.

In particular, in some embodiments, the mammalian milk like product described anywhere herein is extracted, isolated and purified as part of the methods described anywhere herein. Thus, the invention provides an isolated mammalian milk like product, for example an isolated human milk like product.

In some embodiments, the isolated mammalian milk like product is formulated into a composition. In some embodiments, the isolated mammalian milk like product is formulated into a nutritional composition.

In some embodiments, particular bioactives of the mammalian milk like product, selected from the group comprising or consisting of: oligosaccharides, lipids, proteins, exosomes and miRNA, are extracted, isolated and purified from the mammalian milk like product. In particular, exosomes are extracted, isolated and purified from the mammalian milk like product. Thus, the invention provides isolated bioactives of the mammalian milk like product, selected from the group comprising or consisting of: oligosaccharides, lipids, proteins, exosomes and miRNA. In particular, the invention provides isolated exosomes extracted, isolated and purified from the mammalian milk like product, for example isolated human exosomes extracted, isolated and purified from the human milk like product.

In some embodiments, the isolated bioactives of the mammalian milk like product are formulated into a composition. In some embodiments, the isolated bioactives of the mammalian milk like product are formulated into a nutritional composition.

In some embodiments, the isolated exosomes of the mammalian milk like product are formulated into a composition. In some embodiments, the isolated exosomes of the mammalian milk like product are formulated into a nutritional composition.

In some embodiments, step C) comprises purifying exosomes from the human milk like product. In some embodiments, step C) comprises purifying exosomes from the human milk like product to remove impurities, for example by chromatography or filtration or ultracentrifugation, in order to isolate the exosomes. In some embodiments, step C) comprises purifying exosomes from the human milk like product by chromatography in orderto isolate the exosomes. In some embodiments, step C) comprises purifying exosomes from the human milk like product by filtration in order to isolate the exosomes. In some embodiments, step C) comprises purifying exosomes from the human milk like product by ultracentrifugation in order to isolate the exosomes.

In some embodiments, the chromatography is column-based chromatography, for example size inclusion or size exclusion chromatography.

It will be understood that a product obtained from a purified compared to a non-purified protocol will be different in terms of its composition. In particular, the surface composition of the product will be different as components such as proteins that are attached to the surface of a product molecule may be removed during the purification steps. For example, proteins present on the surface of the exosomes may be removed.

In some embodiments, step C) further comprises sterilising the isolated exosomes.

In some embodiments, step C) further comprises storing the isolated exosomes at below freezing, optionally at -80°C.

In some embodiments, step C) comprises formulating the isolated exosomes into a powder form, for example by freeze drying or spray drying, or a liquid form.

In some embodiments, the method further comprises formulating the isolated exosomes with supplementary nutritional ingredients.

In some embodiments, the method further comprises dispensing the isolated exosomes into a container for consumption.

### Human milk like products

### 'Standard' human breast milk like product

In one embodiment of the present invention, the human breast milk like product is a 'standard' human breast milk like product, i.e., comprises the same components as human breast milk of a well-nourished mother.

The benefits of breast feeding are well known in the scientific literature and the possibility to have access to human breast milk like product allows its use for a number of equally well-known health benefits.

In such an embodiment, the human breast milk like product can be used as a substitute of breastfeeding under circumstances where real breastfeeding is not possible.

In such embodiment, the human breast milk like product is intended to be used for example to support longer breastfeeding experience for women who have less milk or who stop to produce milk after 6 months from birth.

Similarly, the human breast milk like product is intended to be used for example to allow breastfeeding even under circumstances where sicknesses compromise real breastfeeding from the mother.

In another embodiment, the human breast milk like product is intended to be used under circumstances whereby breastmilk production would not naturally be initiated, for example if an infant is adopted.

In one embodiment, the human milk like product according to the present invention is not the product of human breast milk lactation as occurring in nature.

In one embodiment, the human breast milk like product is for use in providing optimal nutrition for infant.

In one embodiment, the human breast milk like product is for use in providing healthy growth in infants.

In one embodiment, the human breast milk like product is for use in preventing infection, obesity and promoting immunity development in infants.

In one embodiment, the human breast milk like product is a non-modified human breast milk like product.

In another embodiment, the human breast milk like product is a modified human breast milk like product.

In one embodiment, the human milk like product according to the present invention comprises: proteins, lipids, carbohydrates, vitamins and minerals.

In another embodiment, the human milk like product according to the present invention comprises: proteins, lipids, carbohydrates, vitamins, minerals and bioactives.

In one embodiment, the human milk like product according to the present invention comprises: proteins, lipids (including linoleic acid and alpha-linolenic acid), carbohydrates, Vitamins (including Vitamin A, Vitamin D3, Vitamin E, Vitamin K, Thiamin, Riboflavin, Niacin, Vitamin B6, Vitamin B12, Pantothenic acid, folic acid, Vitamin C and Biotin), minerals (including iron, calcium, phosphorus, magnesium, sodium, chloride, potassium, manganese, iodine, selenium, copper and zinc), choline, myoinositol and L-carnitine.

In a further embodiment, the human milk like product according to the present invention also comprises at least one bioactive selected in the group consisting of: growth factors, cytokines, probiotics, extracellular vesicles (e.g. milk fat globules and or exosomes), bioactives from exosomes (for example miRNA) and secretory IgA.

Such human breast milk like product may be prepared according to the method of the present invention for example by including a step C) of addition of growth factors, cytokines, probiotics, extracellular vesicles (e.g. milk fat globules and or exosomes), bioactives from exosomes (for example miRNA) and secretory IgA.

In one embodiment, the human breast milk like product contains probiotics.

Such human breast milk like product may be prepared according to the method of the present invention for example by including a step C) of addition of probiotics (for example *B.Lactis, B.Infantis, L. Ramnhosus)* which can be obtained from several commercially available sources.

In such embodiment, the human breast milk like product may be used for optimizing gastro intestinal function and/or promoting Immunity.

In one embodiment, the human breast milk like product contains secretory IgA and probiotics.

Such human breast milk like product may be prepared according to the method of the present invention for example by including a step C) of addition of a combination of probiotics and secretory IgA which may be prepared as described for example in patent applications WO2009/156301 and WO2009/156367 which are hereby incorporated by reference. In such embodiment, the human breast milk like product may be used for preventing Immunoglobulin deficiency and/or in the prevention of recurrent infection in infants and young children.

### 'Non-standard' human breast milk like product

In one embodiment of the present invention, the human milk like product can have altered ratios and concentrations of components found naturally in human breast milk of a well-nourished mother. This is referred to herein as a "non-standard milk like product".

In one embodiment, the human milk like product according to the present invention may be selected from the group consisting of a milk fortifier, a supplement, and/or a human breast milk replacer adapted for special purposes.

### Human Milk fortifiers and Human milk bioactive supplements

In one embodiment, the method of the present invention provides for a human breast milk like product which may be used to fortify human breast milk naturally obtained from a nursing mother or to fortify infant formulas.

In another embodiment, the method of the present invention provides for a human breast milk like product which may be used as a supplement for infants or young children in need thereof.

In such embodiments the human breast milk like products may be used for providing healthy growth and/or to reduce the risk of developing a disease typically associated to specific conditions in an infant or young child (such as for example asthma, allergy, cognitive alterations) and /or to promote catch up growth, development of immunity, protection from infections.

Remarkably, the human origin of the constituents (especially bioactive constituents) in such fortifiers or supplements combined with the fact that they are according to the method of the invention, is supposed to provide to such constituents an intact or higher functionality.

The human breast milk like product is preferably intended to be used as a fortifier. Such human breast milk like product intended to be used as a fortifier and may be prepared according to the method of the present invention for example by including a step C) of isolation and/or enrichment of (certain) bioactives from the human breast milk like product obtainable from step B). Such isolation step may be performed via classical fractionation, enrichment and/or purification of the non-modified human breast milk like product obtainable from step B).

The human breast milk like product intended to be used as a supplement may comprise one or more bioactives selected from the group consisting of: human milk oligosaccharides (for example 2FL, 3FL, LNT, LnNT, DiFl, 6SL and /or 3SL), lipids, growth factors (for example epidermal growth factor (EGF), heparin binding epidermal growth factor), cytokines (for example transforming growth factor -beta 2 (TGFbeta-2), IL-1. IL-2, IL-6, IL-10, IL-18, interferon gamma (INF-gamma), TNF-alpha), extracellular vesicles (e.g. milk fat globules and or exosomes), exosome comprising microRNAs and antimicrobial/protecting bioactives (for example IgA, lactoferrin, lysozyme, lactadherine). Such human breast milk like product intended to be used as a supplement may be prepared according to the method of the present invention for example by including a step C) of isolation of the bioactives from the non-modified human breast milk like product obtainable from step B). Such isolation step may be performed via classical fractionation, enrichment and/or purification of the non -modified human breast milk like product obtainable from step B).

In one embodiment, the human breast milk like product is a supplement or milk fortifier which contains fucosylated human milk oligosaccharides, for example 2FL and/or 3FL. Such supplement or milk fortifier is for use in completing the profile of human breast milk of women who do not secrete fucosylated oligosaccharides because of the inactivity of their FUT2 gene.

Such human breast milk like product intended to be used as a fortifier or supplement may be prepared according to the method of the present invention for example by including a step C) of isolation and/or enrichment of fucosylated oligosaccharides (for example 2FL and or 3FL) from the non-modified human breast milk like product obtainable from step B).

In such an embodiment, the human breast milk like product may be used for optimizing gastro intestinal function and/or promoting Immunity.

### Human breast milk like product for infants with genetic diseases

In one embodiment, the human breast milk like product according to the present invention may be adapted to address the specific need of infants who are born with a genetic disease.

### Galactossemia

In such embodiment, the human breast milk like product may be adapted to the needs on infants suffering from Galactossemia. Galactossemia is a rare genetic disease that affects babies' ability to metabolize galactose.

In such embodiment, the human breast milk like product should be deprived of lactose and/or lactose containing saccharides. In such embodiment human breast milk like product may be used for providing healthy growth to the infants affected by galactossemia.

In one embodiment, a human breast milk like product deprived of lactose and/or lactose containing saccharides may be obtained according to the method of the present invention by including a step C) of enzymatic treatment (lactase treatment), or of membrane fractionation and ultrafiltration of the non-modified human breast milk like product obtainable from step B).

### Phenyl Keturonia

In such an embodiment, the human breast milk like product may be adapted to the needs on infants suffering from Phenyl Keturonia (PKU). PKU is due to absent or dysfunctional phenylalanine hydroxylase, which converts phenylalanine to tyrosine. Untreated, it leads to severe mental retardation due to brain toxicity.

In such an embodiment, the human breast milk like product should be deprived or depleted of phenylalanine.

In such an embodiment, the human breast milk like product may be used for providing healthy growth to the infants affected by PKU.

In one embodiment, the human breast milk like product is depleted of phenyl alanine in such a way that phenylalanine content is kept below 20 mg/kg body weight of the subject receiving it.

In one embodiment, a human breast milk like product depleted or deprived of phenylalanine may be obtained according to the method of the present invention by including a step C) of enzymatic treatment (protein hydrolysis) or of filtration of the non -modified human breast milk like product obtainable from step B).

In one embodiment, a human breast milk like product depleted of phenylalanine may be obtained according to the method of the present invention by including a step C) of enzymatic treatment (protein hydrolysis) or of filtration of the non - modified human breast milk like product obtainable from step B).

In another embodiment, a human breast milk like product depleted of phenylalanine may be obtained according to the method of the present invention by providing in step B) a culture medium providing limited or zero amounts of phenylalanine, such as for example a culture medium containing Glycomacropeptide (GMP) from whey.

### Additional Embodiments of the Invention

There is provided:
S1. An *in vitro* method for producing mammalian breast milk exosomes, the method comprising:
   A) Culturing mammary epithelial cells in a culture medium to generate lactocyte mammary-like gland organoids; and
   B) Secreting a mammalian milk like product from said lactocytes, and
   C) Purifying the exosomes from the mammalian milk like product to remove impurities, optionally by chromatography or filtration or ultracentrifugation, in order to isolate the exosomes.
S2. The method of statement 2, wherein the time duration of step A) is no longer than 14 days, optionally is 14 days.
S3. The method of statement 1 or 2, wherein the lactocyte mammary-like gland organoids from step A) express one or more mammary gland positive cell markers, optionally selected from KRT-18 and ER.
S4. The method of any one of statements 1 to 3, wherein the lactocyte mammary-like gland organoids from step A) have increased mRNA expression of one or more milk bioactive markers post-induction compared to pre-induction.
S5. The method of any one of statements 1 to 4, wherein the lactocyte mammary-like gland organoids from step A) have increased mRNA expression of LTF post-induction compared to pre-induction.
S6. The method of any one of statements 1 to 5, wherein the lactocyte mammary-like gland organoids from step A) have increased mRNA expression of MFGE8 post-induction compared to pre-induction.
S7. The method of any one of statements 1 to 6, wherein the culture medium is a MammoCult medium, in an appropriate 3D culture system, for example 3D-suspension condition.
S8. The method of any one of statements 1 to 7, wherein step A) further comprises:
   i) culturing the mammary epithelial cells, and
   ii) inducing milk protein expression.
S9. The method of statement 8, wherein step Ai) is for no more than 7 days, and optionally is for 7 days.
S10. The method of statement 8 or 9, wherein step Aii) is for no more than 7 days, and optionally is for 7 days.
511. The method of any one of statements 1 to 10, wherein step A) further comprises:
   i) culturing the mammary epithelial cells in complete MammoCult medium comprising the basal medium, proliferation supplement and supplemented with heparin, and hydrocortisone for 7 days, and
   ii) inducing milk protein expression by incubating the cells in EpiCultB medium supplemented with EpiCult proliferation supplement, hydrocortisone, insulin, FBS, prolactin, progesterone and β-estradiol for 7 days.
S12. The method of any one of statements 1 to 10, wherein step A) further comprises:
   i) culturing the mammary epithelial cells in MammoCultB medium supplemented with MammoCult proliferation supplement, hydrocortisone and heparin for 7 days, and
   ii) inducing milk protein expression by incubating the in EpiCultB medium supplemented with EpiCult proliferation supplement, hydrocortisone, insulin, FBS, prolactin, progesterone and β-estradiol for 7 days.
S13. The method of any one of statements 1 to 12, wherein step C) further comprises treating the modified milk like product to generate a modified mammalian milk like product.
S14. The method of any one of statements 1 to 13, wherein the mammary epithelial cells are human mammary epithelial cells.
S15. The method of any preceding statement, wherein step C) further comprises formulating the isolated exosomes into a powder form, optionally by spray drying or freeze drying, or liquid form.
S16. The method of any preceding statement, wherein step C) further comprises sterilising the isolated exosomes.
S17. The method of any preceding statement, wherein step C) further comprises storing the isolated exosomes at below freezing, optionally at -80°C.
518. The method of any preceding statement, further comprising formulating the isolated exosomes with supplementary nutritional ingredients.
S19. The method of any preceding statement, wherein the isolated exosomes are dispensed into a container for consumption.
S20. A mammary breast milk exosome product which is obtainable according to the method of any preceding statement, optionally a human breast milk exosome.
S21. A method of producing a mammalian milk fortifier, comprising conducting the method of any one of statements 1 to 19.

It should be appreciated that the various aspects and embodiments of the detailed description as disclosed herein are illustrative of the specific ways to make and use the invention and do not limit the scope of invention when taken into consideration with the claims and the detailed description. It will also be appreciated that features from aspects and embodiments of the invention may be combined with further features from the same or different aspects and embodiments of the invention.

As used in this detailed description and the appended claims, the singular forms "a," "an" and "the" include plural referents unless the context clearly dictates otherwise.

### Figures

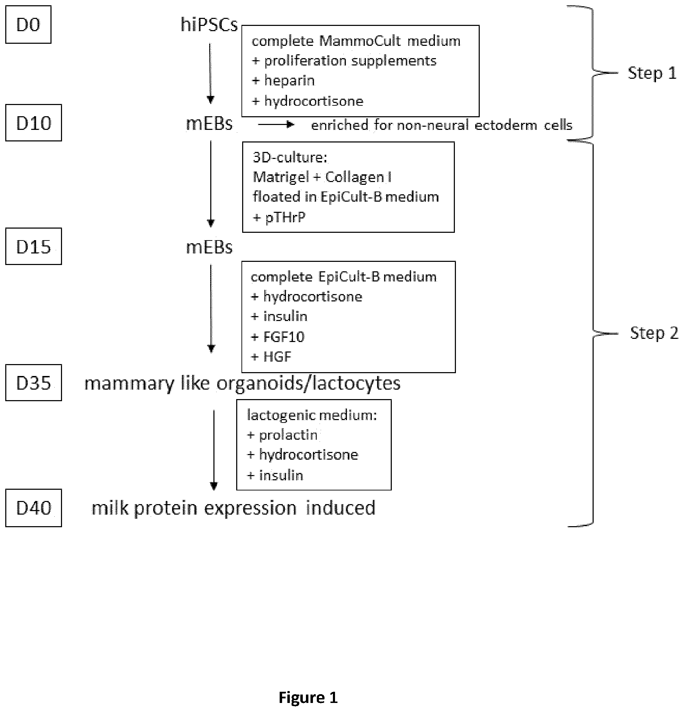

**Fig. 1****:** shows the differentiation of human induced pluripotent stem cells (hiPSCs) according to the protocol outlined in Ying Qu and as applied in one alternative in step A) of the inventive methods.

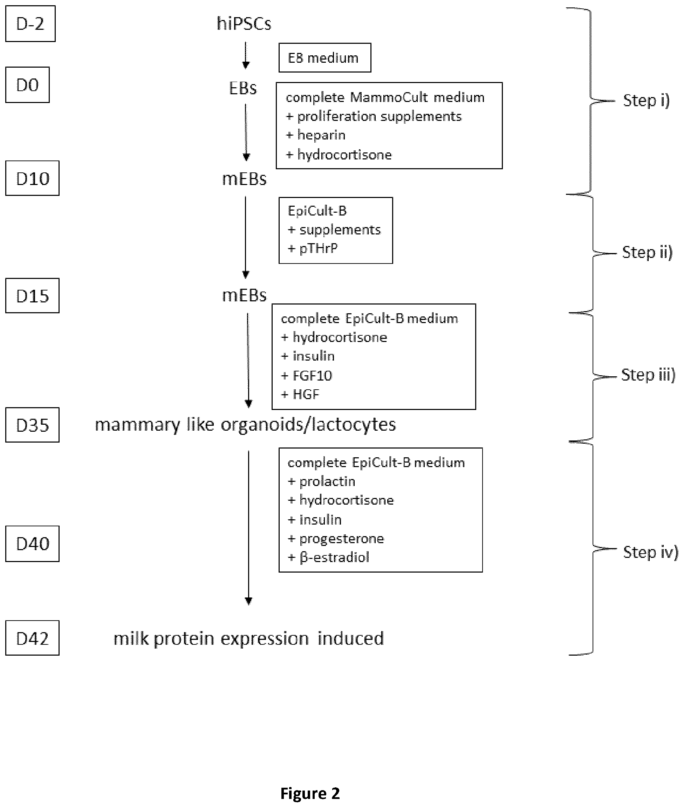

**Fig. 2****:** shows the differentiation of human induced pluripotent stem cells (hiPSCs) according to step A).

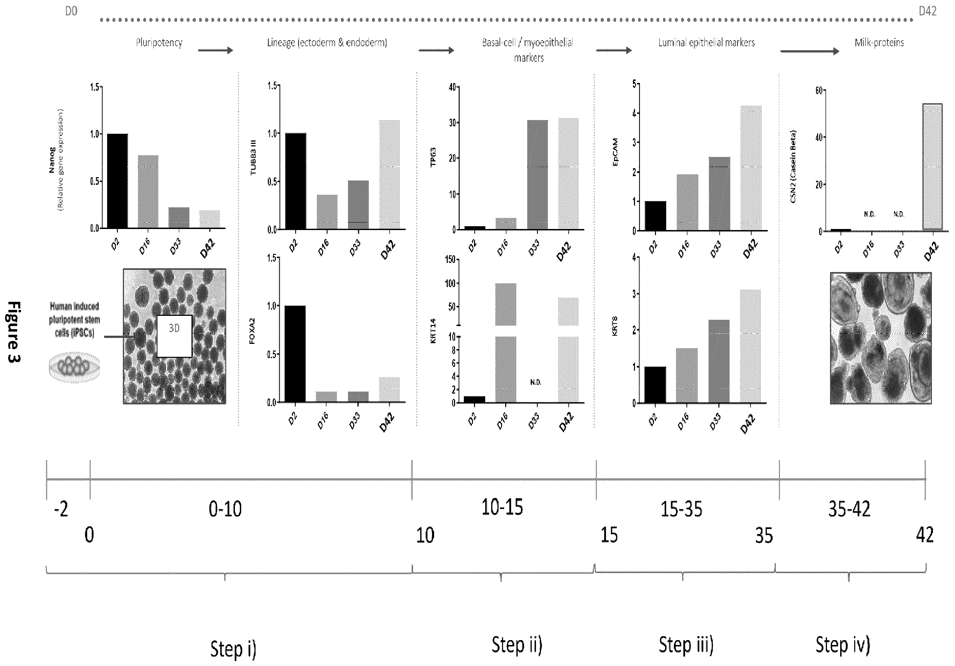

**Fig. 3****:** shows that three-dimensional organotypic cultures of hiPSCs as produced according to the methods of **Fig. 2** are highly permissive for mammary glands specification. mRNA expression of Nanog, TUBB3, FOXA2, TP63, KR-14, EpCAM, KRT8 and CSN2 for 3D-differentiation (42 days) protocol are shown. Markers from left to right: Stages of Pluripotency (Nanog), Lineage (ectoderm & endoderm) (TUBB3, FOXA2), Basal-cell/myoepithelial markers (TP63, KR-14), Luminal epithelial markers (EpCAM, KRT8), and milk proteins (CSN2 (Casein Beta)).

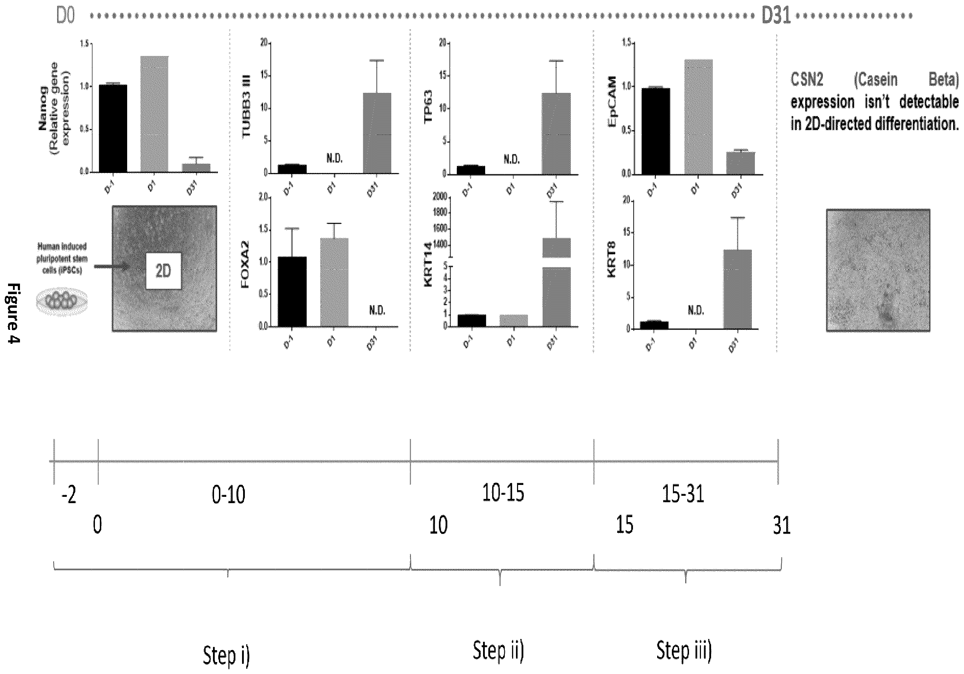

**Fig. 4****:** shows the two-dimensional organotypic culture of hiPSCs produced as a comparative example. mRNA expression of Nanog, TUBB3, FOXA2, TP63, KR-14, EpCAM, KRT8 and CSN2 for 2D-differentiation (31 days) protocols are shown. Markers from left to right: Sages of Pluripotency (Nanog), Lineage (ectoderm & endoderm) (TUBB3, FOXA2), Basal-cell/myoepithelial markers (TP63, KR-14), Luminal epithelial markers (EpCAM, KRT8), and milk proteins (CSN2 (Casein Beta)).

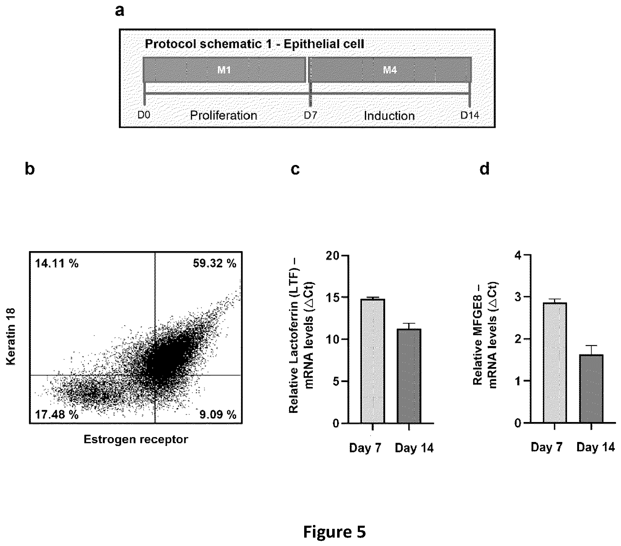

**Fig. 5****:** shows lactation induction in three-dimensional (3D) cultures of mammary epithelial cells. **a,** Schematic illustration of the culture protocol of mammary epithelial cells and lactation induction (D7-14). **b,** Analysis of the mammary gland markers keratin 18 and estrogen receptor by flow cytometry, during the proliferation stage of the mammary gland epithelial cell culture. c, RNA expression (ΔCt) level of human lactoferrin (LTF) and milk fat globule-EGF factor 8 (MFGE8) / lactadherin during the proliferation (Day 7) and lactation induction stages (Day 14). The △Ct method is used in the panel presentations to rank the LTF and MFGE8 genes by calculating the average standard deviation (SD) based on the relative expression of candidate reference gene. The indicated gene with the lowest SD was identified as the most stable or most expressed gene.

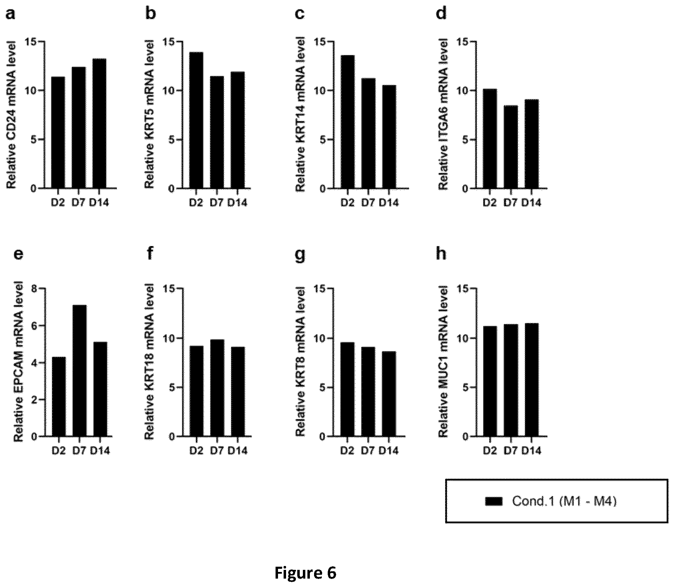

**Fig. 6****:** shows the expression of different mammary epithelium markers in mammary epithelium cells using NanoString technology for gene expression profiling (a**h**).

### Experimental section

### Example 1

### Cultivation and differentiation of hiPSCs into lactocytes to obtain a human milk like product

Lactocytes are cultured starting from ihPSCs according to the procedure described in Ying Qu et al, Stem Cell Report vol 8, 205-215 February 14th 2017 and the human milk like product thereby secreted is collected and can be used in therapy and/or as a breastfeeding substitute according to the present invention.

### Example 2

### Cultivation and differentiation of hiPSCs into 3D-lactocytes to obtain a human milk like product

Lactocytes are cultured starting from hiPSCs according to the method of the present invention following steps A) and B) as described above) and the human milk like product thereby secreted is collected and can be used in therapy and/or as a breastfeeding substitute according to the present invention.

### Example 3

### Alternative methods of cultivation and differentiation of hiPSCs into lactocytes to obtain a human milk like product

Efficient lactocytes differentiation from hiPSCs can be obtained from alternative culture conditions including conditions 1 to 4 as below described:
1. 2D culture on vitronectin coated plates as monolayer of cells derived from the EBs and cultured for at least 28 days in a medium containing (RPMI 1640 with L-glutamine; Fetal bovine serum (FBS); Insulin; Epidermal growth factor (EGF); hydrocortisone; Pen-Strep (penicillin/streptomycin : antibiotic-antimycotic solution).
2. 2D culture on vitronectin coated plates of attached aggregates (EBs) of cells derived from the EBs and cultured for at least 28 days in a medium containing (RPMI 1640 with L-glutamine; Fetal bovine serum (FBS); Insulin; Epidermal growth factor (EGF); hydrocortisone; Pen-Strep (antibiotic-antimycotic solution).
3. 3D culture in suspension in MammoCult medium for at least 10 days and then culture in mixed floating gels (for example Matrigel and Collagen 1) for another 5 days in a specific medium (for example EpiCultB) in presence of Parathyroid hormone followed by 25 days in presence of insulin, HGF, hydrocortisone and FGF10;
4. 3D culture of EBs in suspension (ultra low adherent plate) in MammoCult medium for at least 10 days and then in suspension culture for another 5 days in a specific medium (for example EpiCultB) in presence of Parathyroid hormone followed by 25 days in presence of insulin, HGF, hydrocortisone and FGF10.

### Example 4

### 2D- and 3D-lactocyte differentiation based on human-induced pluripotent stem cell (hiPSC) line 603

### (a) 3D-lactocyte differentiation based on human-induced pluripotent stem cell (hiPSC) line 603:

The human-induced pluripotent stem cell (hiPSC) line 603 was used for 3D-lactocyte differentiation. The human-induced pluripotent stem cell (hiPSC) line 603was purchased from Fujifilm Cellular Dynamics, Inc (FCDI).
(i) For the 3D differentiation protocol (according to the invention), EBs (spheroids) were formed by incubating single cells of hiPSC in E8 medium with 10uM rock inhibitor at 37°C, 5% CO2 in rotation at 95 rpm overnight.

Second day, medium was replaced with E8 (day -2-day 0).

Next day, medium was replaced with Mammo1 medium (MammoCult - medium with proliferation supplements, heparin (4µg/mL), and hydrocortisone (0.48µg/mL) with penicillin/streptomycin) for 10 days (day 0-day 10). Medium was changed every second day.
(ii) The differentiation was followed by 5 days in Mammo2 medium (EpiCultB + supplements, pTHrP 100ng/ml plus penicillin/streptomycin). Culture medium was changed every 3 days (day 10-day 15).
(iii) In order to induce branching epithelial structure,alveolar differentiation and mammary cell specification, mEBs (spheroids/mammospheres) were fed with Mammo3 medium (complete EpiCultB, hydrocortisone (1 µg/ml), insulin (10 µg/ml), FGF10 (50 ng/ml), HGF (50 ng/ml) and penicillin/streptomycin) for 20 days. Medium was changed every 3 days (day 15-day 35).
(iv) Finally, to induce the milk bioactive production (3D), we used the Mammo4 medium (complete EpiCultB, 10% FBS, prolactin (10 µg/ml), hydrocortisone (1 µg/ml), insulin (10 µg/ml), progesterone, β-estradiol and penicillin/streptomycin for 7 days and medium was changed every 3 days (day 35-day 42). During all the differentiation procedure, spheroids were maintained in the suspension culture (rotating at 95 rpm). The differentiation procedure ended at day 42. Results are displayed in Figure 3.

### (b) 2D-lactocyte differentiation based on human-induced pluripotent stem cell (hiPSC) line 603

The human-induced pluripotent stem cell (hiPSC) line 603 was used also for 2D-lactocyte differentiation. The human-induced pluripotent stem cell (hiPSC) line 603was purchased from Fujifilm Cellular Dynamics, Inc (FCDI).

For the 2D-differentiation protocol (used for comparison), we used the Lacto medium during all the differentiation stages (RPMI 1640, 20% FBS, 1mM glutamine, 4 µg/ml insulin, 20 ng/ml EGF, 0.5µg/ml hydrocortisone with penicillin/streptomycin). Cells were incubated at 37°C, 5% CO2. Medium was replaced every second day. Results are displayed in Figure 4.

### (c) Results

The different differentiation stages during lactocyte derivation were captured using quantitative RT-PCR (Figure 3, 3D-differentiation, Figure 4, 2D-differentiation). In both 2D- and 3D-settings, NaNog expression as a marker for pluripotency is decreased while cells are passing towards the maturation and differentiation. The neuroectodermal and endodermal markers, TUBB3 (Tubulin Beta 3 Class III) and Forkhead box protein A2 (FOXA2) were not expressed significantly in 3D-format and TUBB3 elevation is only captured in 2D-setting. This demonstrates that hiPSCs are patterned towards the non-neural ectodermal lineage, thus enriching mammary progenitors in 3D-format. We investigated the expression pattern of commonly used basal cell/myoepithelial markers, such as p63 (a p53-homologous nuclear protein) and cytokeratin 14 (KRT-14). Both markers are detectable significantly in both systems. Additionally, the epithelial cell adhesion molecule (EpCAM) and cytokeratin 8 (KRT8) were tracked only in the 3D-system and KRT8 was only partially expressed in the2D-format. Consequently, 3D-platfrom in an organotypic setting expressed common breast tissue, luminal, and basal markers. Such mammary like organoids express human breast specific proteins including CSN2 (casein beta), milk protein peptides, and hormone receptors. The luminal cells specifically express EpCAM, MUC1, CD49F, GATA3, CK8, and CK18 while basal cells will specifically express CK14, α-smoothmuscle actin and P63. Eventually EpCAM and CD49F double positive cells can be detected at an earlier progenitor stage between D10 and D35. Interestingly, CSN2 expression is only captured at the last time point (D42) of the 3D-organotypic system and not in the 2D-directed differentiation platform.

Analysis of the mammary like organoids secretome showed secretion of human milk specific bioactives including oligosaccharides (including lactose and some HMOs), lipids (including 4 fatty acids), proteins (7 detected including caseins), and miRNA (75 detected, including 11 typically detected in HBM) as below described.

Primary cell supernatant was analyzed for presence of lactose or human milk oligosaccharides following the procedure described in "Austin and Benet, Quantitative determination of non-lactose milk oligosaccharides, Analytica Chimica Acta 2018, 1010, 86-96" with minor modification. The samples were analysed with UHPLC and detected lactose or human milk oligosaccharides (HMOs) were quantified against a calibration curve of lactose and a mix of 7 HMOs (2'FL, 3FL, DFL, LNT, LNnT, 3'SL and 6'SL). The method had an estimated limit of 0.1mg/L. In the primary cell supernatants, Lactose (0.22 mg/l) and 6'SL (0.32 mg/l) were detected at day 42.

Fatty acids were analysed in media and cell supernatants by gas chromatography coupled with flame ionization detector. Briefly, the supernatants obtained at day 42 is analysed to investigate the presence of fatty acids contained in several lipid classes. A 7890A gas-chromatograph with a 7693 autosampler with preparative station module equipped with a fused-silica CP-Sil 88 capillary column (100% cyanopropylpolysiloxane; 100 m, 0.25 mm id, 0.25 mm film thickness is used with a split injector (1:25 ratio) heated at 250°C and a flame-ionization detector operated at 300°C. Preparation of FAMEs (fatty acids methyl esters) is performed by direct transesterification of sample with methanolic chloridric acid. Separation of FAMEs is performed using capillary gas chromatography-FID (GC). Identification of FAMEs is done by retention time (RT) and comparison with an external standard. Quantification of fatty acids is done by calculation using methyl C11:0 as internal standard. Transesterification performance of the method is controlled with TAG C13:0 as second internal standard. After addition of internal standards, the solution was mixed with 2 mL of methanol, 2 mL of Methanol/HCl (3N) and 1 mL of hexane. After heating at 100°C/60min, the sample is cooled down to room temperature (about 15 min) and the reaction is stopped by adding 2mL of water. After centrifugation the organic phase is directly injected into the GC.

Fatty acid results from protocol of Example 4a at time day 42 are reported in table 1 (differences observed between media and supernatant).

**The table 1 below lists the expressed fatty acids in cell supernatant sample.**

| **Fatty acid** | **Detected amount in cell supernatant (mg/100 mL)** |
|---|---|
| C-4:0 | 2.53 |
| C-8:0 | 0.49 |
| C-10:0 | 0.38 |
| C-14:0 | 0.44 |
| C-15:0 | 0.41 |
| C-16:0 | 1.85 |
| C-16:1n7 | 0.08 |
| C-17:0 | 0.09 |
| C-18:0 | 0.97 |
| C-18:1 n9 | 18.82 |
| C-18:1 | 0.28 |
| C-18:2 n6 | 2.16 |
| C-20:0 | 0.13 |
| C-20:1 n9 | 0.11 |
| C-18:3 n3 | 0.08 |
| C-22:0 | 0.29 |
| Other fatty acids | 1.38 |

Proteins in the cell supernatant were analysed using SDS-PAGE profiling and then band isolation for identity confirmation by LC-MSMS. For SDS-PAGE analysis, the total volume of the prepared sample was loaded on the gel. A human milk sample was added for comparison as control. Selected gel regions (bands) were cut to look for human proteins by LC-MSMS. Eventually, bands were submitted to in-gel trypsin digestion and analyzed by LC-MSMS. LC-MSMS data were analyzed with Peaks Studio and matched against the UniProt database for human proteins.

**The table 2 below lists the best candidates for all the excised bands.**

| **Name of expressed proteins in the cell supernatant** |
|---|
| Lactoferrin |
| Albumin |
| Prolactin |
| Alpha S1-casein |
| Hemoglobin subunit beta |
| Hemoglobin subunit alpha |
| α-lactalbumin |
| Alpha-2-macroglobulin |
| β-casein |
| bile salt-activated lipase |
| κ-casein |
| lactadherin |
| CD14 |
| fatty acid synthase |
| IgA |
| plgR |
| Serum albumin |
| Xanthine dehydrogenase |

Exosome isolation and miRNA profiling was performed using ExoQuick polymer nets. ExoQuick polymer works to precipitate exosomes by forming a network and collects all exosomes of a certain size. Once the ExoQuick mesh is formed, a simple, low-speed centrifugation easily precipitates the exosomes as a pellet. The exosomes are intact, ready for protein or RNA analysis and are bioactive for functional studies. Precipitation buffer was added in a ration 0.25X to the sample then vortex. The mix was incubated overnight at 4°c. After incubation, samples were centrifuged 30 min at 1,500xg. The exosome pellet was re-suspended by vertexing in initial volume with Buffer XE (QIAGEN) for QC or Lysis Buffer from HTG EdgeSeq miRNA Whole Transcriptome Assay for miRNA profiling. In order to assess the extracellular vesicles (EVs) isolation, the supernatant was first centrifuged at 3000g for 15 min to remove cell pellet and debris. Then 100 microliters of media was used for an overnight precipitation at 4°c with ExoQuick buffer (ratio 0.25X). EV precipitates were recovered by centrifugation for 30 min at 1500g. Two precipitations were performed for each sample, one EV precipitation was resuspended in Buffer XE (QIAGEN) for potential further analysis, and a second one in only 50 ul HTG Lysis buffer in order to concentrate by 10-fold before miRNA profiling with HTG.

For miRNA profiling, samples were used directly in the first step of lysis. Thus, Whole sample was used directly and was lysed with Plasma lysis buffer in a ratio1:1. Next, proteinase K (1/10) was added and the samples were incubated 3h at 50°c at 600rpm on Thermomixer. EVs were resuspended in Lysis buffer and lysed in the same conditions, with an incubation step at 95°c for 10min added before the lysis incubation. 26 µl of lysate was process with 70 µl of oil on the HTG processor following the HTG EdgeSeq miRNA Whole Transcriptome Assay V2 procedure. For indexing and amplification libraries, samples were tagged with Illumina adaptors and indexes by PCR with OneTaq^{®} Hot Start 2X Master Mix GC Buffer (95°C - 4 min; 16 cycles: 95°C-15 sec, 56°C-45 sec, 68°C- 45 sec; 68°C10 min; Hold at 4°C) and AMPure cleaned (ratio 2.5) on a robotic liquid handler SciClone NGS WorkStation (Perkin Elmer). Pools were obtained with our custom pooling program on Hamilton robot. The samples were pooled based on GX touch Chip HS quantification. The pools were purified manually a second time with AMPure Bead (ratio 1.8) to remove potential remaining traces of primer-dimer and quantified with Qubit to adjust the final concentration to 2 nM. And as a last step, for MiSeq sequencing, pools were loaded on MiSeq at 20pM with a 5% PhiX spike and sequenced for 50 base Single read on MiSeq with 150V3 kit.

Briefly, 974 miRNAs detected in the in the cell supernatant which more than 75 of them are highly expressed miRNAs in the milk samples.

**The table 3 below lists the top ten highly expressed miRNAs.**

| **miRNA name** | **log2 counts** | **CV** |
|---|---|---|
| miR-21-5p | 9.76 | 0.01 |
| miR-181a-5p | 9.07 | 0.03 |
| miR-30d-5p | 8.63 | 0.01 |
| miR-30b-5p | 8.63 | 0.01 |
| miR-22-3p | 8.49 | 0.01 |
| miR-146b-3p | 8.40 | 0.01 |
| miR-30c-5p | 8.12 | 0.04 |
| miR-30a-5p | 7.63 | 0.02 |
| miR-30e-5p | 7.26 | 0.01 |
| miR-148b-3p | 6.77 | 0.04 |

Our findings provide a novel iPSC-based 3D-organotypic model for studying the regulation and development of normal mammary cell fate and function as well as breast milk bioactives production.

### Example 5

Mammary epithelial cells were cultivated in 3D-formats with and without matrixes for 7 days proliferation and 7 days induction stages using differentiation media M1/M4 (Figure 5a). Other differentiation media may also be used. Briefly, 1-5 million dissociated epithelial cells were plated in the 6 well-plates (ultra-low attachment) containing 4.5 mL media using a planar shaker platform. Cultured cells can express mammary-gland specific markers such as keratin 18 and estrogen receptor using flow cytometry quantification (Figure 5b) and are able to express mRNA-level of human lactoferrin (LTF) and milk fat globule-EGF factor 8 (MFGE8) or lactadherin during the proliferation (Day 7) and induction period (Day 14) (Figure 5c, d).

Using NanoString technology for gene expression profiling, we assessed the expression of different mammary epithelium markers in mammary epithelium cells (Figure 6). There was a consistent expression of the mammary epithelial progenitor marker CD24, with a tendency for higher expression during lactation (Figure 6a). It is interesting to observe that cells show a decrease in mammary basal-like cell's marker expression such as KRT5, KRT14 and ITGA6 (CD49f) as lactation progresses (Figure 6b-d). It appears that cells maintained a luminal-like phenotype during and after lactation using different markers, such as EpCAM, KRT8, KRT18 and MUC1 (Figure 6e-h). The post-induction period is characterized by the initiation of lactocyte (luminal-like cells) specific secretory profiles for different proteins, such as lactoferrin (LTF), clusterin and fatty acid synthase (FASN) (Table 1a). Exosomes isolated from purified epithelial cells after lactation also show abundant expression of milk-specific miRNAs, which is listed in Table 1b. Expression of mammary epithelium markers in mammary epithelium cells and milk-specific miRNA in exosomes purified epithelial cells after lactation was also observed using alternative differentiation conditions.

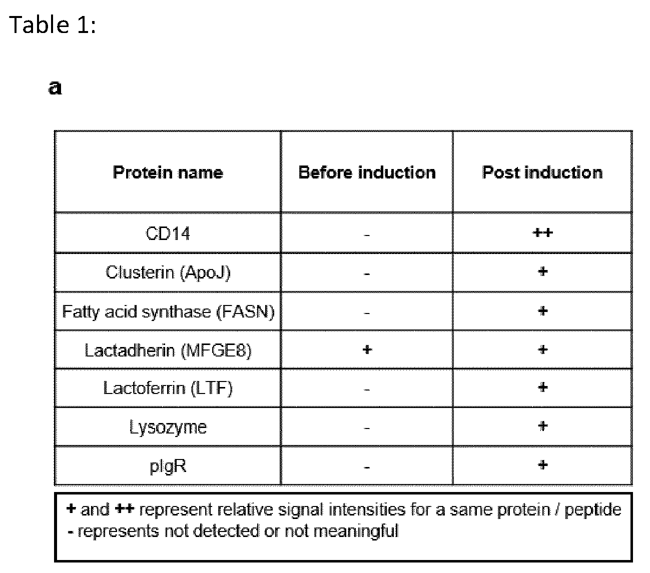

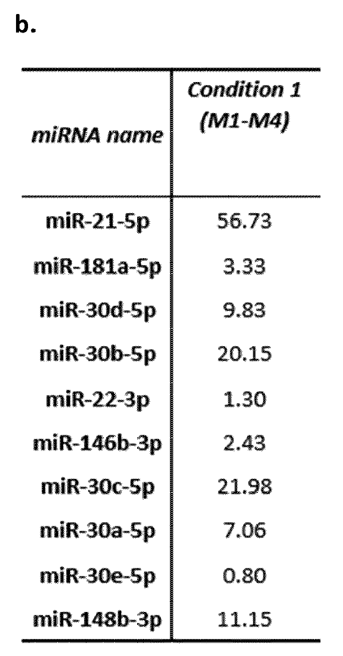

It should be understood that various changes and modifications to the presently preferred embodiments described herein will be apparent to those skilled in the art.

Such changes and modifications can be made without departing from the spirit and scope of the present invention and without diminishing its attendant advantages. It is therefore intended that such changes and modifications be covered by the appended claims.

## Claims

1. An *in vitro* method for producing mammalian breast milk exosomes, the method comprising:
A) Culturing mammary epithelial cells in a culture medium to generate lactocyte mammary-like gland organoids,
B) Secreting a mammalian milk like product from said lactocytes, and
C) Purifying the exosomes from the mammalian milk like product to remove impurities, optionally by chromatography or filtration or ultracentrifugation, in order to isolate the exosomes.

2. The method of claim 1, wherein the time duration of step A) is no longer than 14 days, optionally is 14 days.

3. The method of claim 1 or 2, wherein the lactocyte mammary-like gland organoids from step A) express one or more mammary gland positive cell markers, optionally selected from KRT-18 and ER.

4. The method of any one of claims 1 to 3, wherein the lactocyte mammary-like gland organoids from step A) have increased mRNA expression of one or more milk bioactive markers post-induction compared to pre-induction.

5. The method of any one of claims 1 to 4, wherein the lactocyte mammary-like gland organoids from step A) have increased mRNA expression of LTF post-induction compared to pre-induction.

6. The method of any one of claims 1 to 5, wherein the lactocyte mammary-like gland organoids from step A) have increased mRNA expression of MFGE8 post-induction compared to pre-induction.

7. The method of any one of claims 1 to 6, wherein the culture medium is a MammoCult medium, in an appropriate 3D culture system, for example 3D-suspension condition.

8. The method of any one of claims 1 to 7, wherein step A) further comprises:
i) culturing the mammary epithelial cells, and
ii) inducing milk protein expression.

9. The method of claim 8, wherein step Ai) is for no more than 7 days, and optionally is for 7 days.

10. The method of claim 8 or 9, wherein step Aii) is for no more than 7 days, and optionally is for 7 days.

11. The method of any one of claims 1 to 10, wherein step A) further comprises:
i) culturing the mammary epithelial cells in complete MammoCult medium comprising the basal medium, proliferation supplement and supplemented with heparin, and hydrocortisone for 7 days, and
ii) inducing milk protein expression by incubating the cells in EpiCultB medium supplemented with EpiCult proliferation supplement, hydrocortisone, insulin, FBS, prolactin, progesterone and β-estradiol for 7 days.

12. The method of any one of claims 1 to 10, wherein step A) further comprises:
i) culturing the mammary epithelial cells in MammoCultB medium supplemented with MammoCult proliferation supplement, hydrocortisone and heparin for 7 days, and
ii) inducing milk protein expression by incubating the in EpiCultB medium supplemented with EpiCult proliferation supplement, hydrocortisone, insulin, FBS, prolactin, progesterone and β-estradiol for 7 days.

13. A method of any one of claims 1 to 12, wherein step C) further comprises treating the modified milk like product to generate a modified mammalian milk like product.

14. The method of any one of claims 1 to 13, wherein the mammary epithelial cells are human mammary epithelial cells.

15. The method of any preceding claim, wherein step C) further comprises formulating the isolated exosomes into a powder form, optionally by spray drying or freeze drying, or liquid form.

16. The method of any preceding claim, wherein step C) further comprises sterilising the isolated exosomes.

17. The method of any preceding claim, wherein step C) further comprises storing the isolated exosomes at below freezing, optionally at -80°C.

18. The method of any preceding claim, further comprising formulating the isolated exosomes with supplementary nutritional ingredients.

19. The method of any preceding claim, wherein the isolated exosomes are dispensed into a container for consumption.

20. A mammary breast milk exosome product which is obtainable according to the method of any preceding claim, optionally a human breast milk exosome.

21. A method of producing a mammalian milk fortifier, comprising conducting the method of any one of claims 1 to 19.
